(19) Europäisches Patentamt
European Patent Office
Office européen des brevets

(11) **EP 4 051 083 B1**

(12) **EUROPEAN PATENT SPECIFICATION**

(45) Date of publication and mention
of the grant of the patent:
**11.02.2026 Bulletin 2026/07**

(21) Application number: **20700708.9**

(22) Date of filing: **13.01.2020**

(51) International Patent Classification (IPC):
*A61B 3/00* *(2006.01)*    *A61B 3/135* *(2006.01)*
*A61B 3/14* *(2006.01)*

(52) Cooperative Patent Classification (CPC):
**A61B 3/0008; A61B 3/135; A61B 3/14**

(86) International application number:
**PCT/EP2020/050728**

(87) International publication number:
**WO 2021/144001 (22.07.2021 Gazette 2021/29)**

(54) **OPHTHALMOLOGIC MICROSCOPE WITH SYNCHRONIZED LIGHT SOURCE AND CAMERA**

OPHTHALMOLOGISCHES MIKROSKOP MIT SYNCHRONISIERTER LICHTQUELLE UND KAMERA

MICROSCOPE OPHTALMOLOGIQUE AVEC SOURCE DE LUMIÈRE SYNCHRONISÉE ET CAMÉRA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.09.2022 Bulletin 2022/36**

(73) Proprietor: **HAAG-STREIT AG**
**3098 Köniz (CH)**

(72) Inventors:
• **TRITTIBACH, Caspar**
**3012 Bern (CH)**
• **ZUMKEHR, Frank**
**3052 Zollikofen (CH)**

(74) Representative: **E. Blum & Co. AG**
**Franklinturm**
**Hofwiesenstrasse 349**
**8050 Zürich (CH)**

(56) References cited:
GB-A- 2 425 186    JP-A- 2004 255 177
US-A- 6 072 623    US-A1- 2005 195 360

**Description**

<u>Technical Field</u>

The invention relates to ophthalmologic microscopes, in particular slit lamp microscopes, having an illumination device adapted to generate illumination pulses on the eye and a microscope device with a camera for recording images of the eye. The invention also relates to methods for operating such a device.

<u>Background Art</u>

EP 2549913 describes an ophthalmologic microscope having an illumination device generating illumination pulses as well as a microscope having a camera. The pulsed light and the camera are synchronized in order to have a defined illumination while recording a camera frame.

GP2425186A describes a slit lamp microscope having a camera with a flash as well as a pulsed light source. The flashing of the camera is detected to synchronize the pulsed light source with the shutter opening of the camera.

US6072623 describes a slit lamp microscope also having a pulsed light source. The pulsing of the light source is synchronized with an image signal from the camera.

JP 2004-255177 A discloses an ophthalmologic microscope with an LCD array comprising switchable liquid crystal elements for selective light transmission (¶0041), a CCD camera for image capture (¶[0038]), and a controller for image analysis and optimization of red reflex illumination (10038).

<u>Disclosure of the Invention</u>

The problem to be solved by the present invention is to provide an improved control for such a microscope for better image quality.

This problem is solved by the devices and methods according to the independent claims.

Accordingly, the invention relates to ophthalmologic microscopes comprising at least the following elements:

i) An illumination device generating illumination pulses: The illumination device is adapted and structured to generate individual light pulses for illuminating the eye to be examined. It comprises at least the following elements:

a) At least one light source: This is the part of the illumination device that generates the light.
b) A spatial light modulator: This part is structured to spatially modulate the light. It is an electronically controlled light modulator.
c) Illumination imaging optics: This part is structured to project the light onto the eye. It projects an image of the spatial light modu-
lator onto the eye, thereby generating a defined illumination pattern on the eye.

ii) A microscope device: The microscope device is adapted to record an image of the eye. It comprises at least the following elements:

a) Microscope optics: The microscope optics generates an image of the eye.
b) At least one electronic camera: The camera is adapted to record the projected image from the microscope optics.

iii) A control unit: The control unit controls the operation of the elements of the device.

According to the invention, the camera has a frame signal output carrying a signal indicative of the times when the camera records a frame. In particular, it may generate a single pulse synchronized to each frame it takes, i.e. synchronized to the integration phase of that frame.

The control unit is structured to synchronize the illumination device to this frame signal output. This allows to run the camera independently, e.g. in its native free-running mode, which allows for a faster frame rate. Hence, in this configuration, the camera acts master device while the illumination device acts as a slave device synchronized to the timing of the master device.

In a preferred aspect of the invention, the control unit is structured to generate at least two illumination pulses on the eye for each frame recorded by the camera. This has the advantage that the light pulse rate that the eye sees is higher than the frame rate of the camera, thereby reducing the risk of irritating the patient with flicker effects.

The invention also relates to methods for operating ophthalmologic microscopes, in particular the microscopes as mentioned above. Same as above, the microscope comprises at least the following elements:

i) An illumination device generating illumination pulses: The illumination device is adapted and structured to generate individual light pulses for illuminating the eye to be examined. It comprises at least the following elements:

a) At least one light source: This is the part of the illumination device that generates the light.
b) A spatial light modulator: This part is structured to spatially modulate the light. It is an electronically controlled light modulator.
c) Illumination imaging optics: This part is structured to project the light onto the eye. It projects an image of the spatial light modulator onto the eye, thereby generating a defined illumination pattern on the eye.

ii) A microscope device: The microscope device is adapted to record an image of the eye. It comprises at least the following elements:

a) Microscope optics: The microscope optics generates an image of the eye.
b) At least one electronic camera: The camera is adapted to record the projected image from the microscope optics.

iii) A control unit: The control unit controls the operation of the elements of the device.

The method comprises the step of sequentially recording frames by means of the camera.

According to a preferred embodiment, the method comprises the steps of generating, by means of the illumination device, light pulses synchronized to the frame signal output of the camera.

According to this preferred embodiment of the invention, the method further comprises the step of generating, by means of the illumination device, at least two illumination pulses for each frame recorded by the camera.

Advantageously, the camera is run in its "free-running mode". In the free-running mode, the camera is not recording its frames synchronized with an external trigger signal but rather records a next frame as soon as it has finished recording and processing the previous frame. In free-running mode, cameras can reach a higher frame rate than when they are triggered to record the frames by means of an external source.

According to the invention, the spatial light modulator is an electronically controlled spatial light modulator comprising a two-dimensional array of individually controllable pixels, which are synchronized, by the control unit of the device, to the frame signal output of the camera.

In one embodiment, the spatial light modulator may comprise a two-dimensional array of micro-mirrors that are individually deflectable into a first and a second position. The first position may e.g. correspond to the on-state and the second position may e.g. correspond to the off-state of the pixel. In this context, a micro-mirror is advantageously a mirror having an area of less than 1 mm$^2$, in particular less than 100 $\mu$m$^2$.

The light source is pulsed, i.e. the control unit is structured to pulse the light source. This is done in a manner synchronized to the frame signal output of the camera.

If the microscope uses a pulsed illumination as well as a light modulator having an array of individually controllable pixels, the control unit may be adapted to:

- bringing the pixels into a given configuration during a dark phase prior to a given light pulse, and
- starting the light pulse only when the pixels are in the desired configuration.

In this case, the dark phase is before a light pulse is exploited for setting up the light modulator. This setting up may take some time, e.g. because the pixels are slow or because it takes time to feed the pattern information sequentially into the light modulator, and it is therefore best done before the light pulse.

The method or control unit may be adapted to perform at least the following two steps:

- Predicting the time when the camera will record a next frame: This prediction is made depending on the time when the camera recorded at least one previous frame. In particular, it may depend on the times when the camera recorded several previous frames.
- Preparing at least part of the illumination device before the predicted time for recording the next frame.

This allows to set up at least part of the illumination optics, e.g. the spatial light modulator, before a frame is recorded, thereby allowing for a more defined image recording. This is particularly advantageous in the first aspect of the invention.

The device may further comprise several light sources and at least one light detector adapted to measure the light intensity at a location between the light sources and the spatial light modulator, i.e. the brightness of the light source independently of the state of the light modulator. In that case, the method or control unit may be adapted to perform at least the following two steps:

- Bringing the light modulator into a non-transmitting mode: In this mode, no light is projected onto the eye even if the light source is switched on.
- While the light modulator is in this non-transmitting mode, pulsing one light source for measuring the brightness of this light source.

This allows to intermittently - in particular while recording of a series of image frames - control the current brightness of the light source. This information can then be used e.g. for adjusting the current through the light source during light pulses while recording the frames on the on-time of the pixels of the light modulator while recording the frames in order to achieve a defined illumination.

Brief Description of the Drawings

The invention will be better understood and objects other than those set forth above will become apparent when consideration is given to the following detailed description thereof. Such description makes reference to the annexed drawings, wherein:

Fig. 1 shows an embodiment of an ophthalmologic microscope,
Fig. 2 shows an embodiment of the components of the illumination device,
Fig. 3 is a schematic representation of micro-mirror-based spatial light modulator, and
Fig. 4 shows an embodiment of a timing diagram for various components of the device.

Modes for Carrying Out the Invention

*Overview*

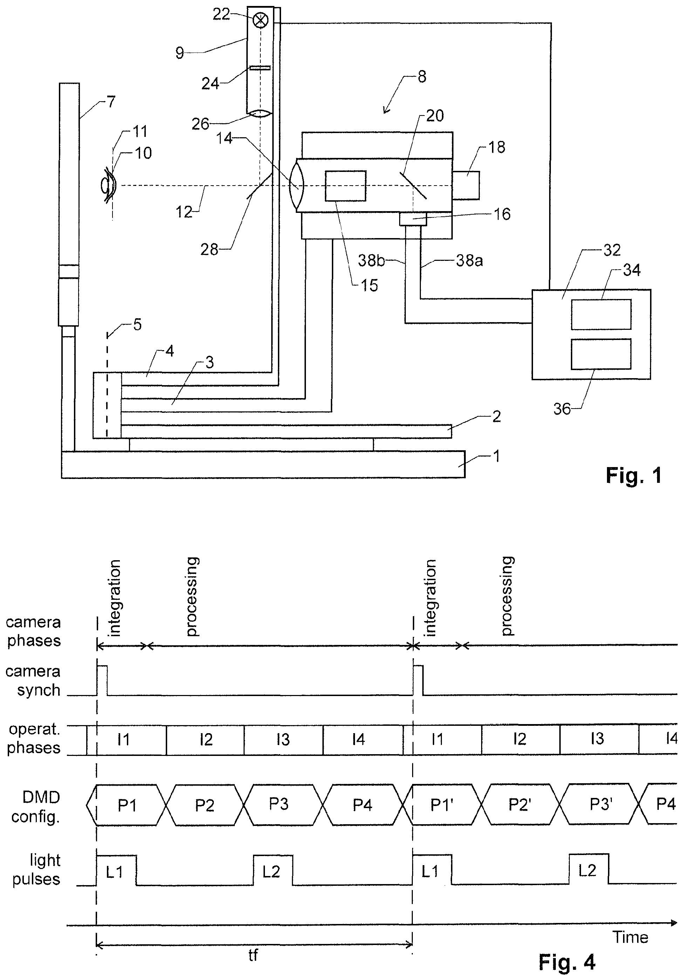

Fig. 1 shows an embodiment of an ophthalmologic microscope, in particular a slit lamp microscope.
The microscope has a base 1 resting e.g. on a desk, a translationally displaceable stage 2 mounted to base 1, a first arm 3, and a second arm 4.
Stage 2 can be linearly displaced along horizontal directions x and z in respect to base 1.
The arms 3 and 4 are mounted to stage 2 and pivotal about a common vertical pivot axis 5, i.e. an axis parallel to vertical direction y.
The device may further include a headrest 7 mounted to base 1 for receiving the patient's head.
Arm 3 carries a microscope device 8, and arm 4 carries an illumination device 9, such as a slit lamp.
Microscope device 8 has an optical axis 12. It comprises microscope optics 14, 15, such as an objective 14 and zoom optics 15, which project an image of eye 10 onto a camera 16 and, optionally, an eyepiece 18. A beam splitter 20 may be provided to spilt light between these components.
Illumination device 9 is adapted to project a structured light beam onto the eye 10 to be examined. It comprises a light source 22, a spatial light modulator 24, and illumination imaging optics 26.
Light source 22 can e.g. comprise several units emitting different wavelengths, e.g. in the red, green, blue, and infrared range of the optical spectrum. These units can be controlled separately in order to change the color of light source 22.
Advantageously, the light source 22 comprises at least one LED and/or semiconductor laser. This type of light sources are advantageous because LEDs and semiconductor lasers can be pulsed rapidly and precisely.
Illumination imaging optics 26 projects the light from modulator 24 onto e.g. the anterior surface of eye 10, e.g. via a mirror 28 mounted to arm 4. The anterior surface of eye 10 is assumed to be located at a target plane 11, which is the optically conjugate plane of spatial light modulator 24 in respect to illumination imaging optics 26.
Illumination device 9 can be arranged above or below mirror 28.
A control unit 32 controls the components of the microscope. In particular, it may e.g. comprise a microprocessor 34 and a memory 36. Microprocessor 34 is programmed to carry out the steps of the method as described below, and memory 36 contains the data and/or instructions to do so.
Camera 16 has is adapted to record a series of individual frames of the image projected onto it.
It has a frame signal output 38a carrying a signal indicative of the time when a frame is recorded. It also has a data output 38b for transferring the recorded image. Both outputs 38a, 38b may be connected to control unit 32.
Camera 16 may e.g. by a CCD camera or any other semiconductor-based camera, such as a camera of the IMX series by Sony.

In semiconductor cameras, the process of recording a frame involves the integration of the optical signal over a certain period. At the end of the integration period, the image can be read out.

The signal at frame signal output 38a is in synch with this integration phase and, depending on camera type, it may e.g. be issued at the beginning or the end of the integration phase.

*Illumination Device*

Fig. 2 shows a more detailed embodiment of illumination device 9. It is designed to project an illumination field of defined, sharp contours onto target, such as the eye 10. The illumination field may e.g. be round, rectangular, or slit-shaped. Even though the illumination device is called a "slit-lamp" herein, the illumination field does not need to be slit-shaped at all. It can take any shape.

In the present embodiment, illumination device 9 comprises four light sources 22a - 22d of different spectral emission characteristics. For example, they may include an infrared light source, a red light source, a green light source, and a blue light source. Advantageously, the light sources are LEDs. In particular, each light source may be a single LED.

The light from each light source is substantially collimated by means of collimation optics 40a - 40d.

Three dichroic mirrors 42a, 42b, 42c are used to combine the light from the light sources 22a - 22d to become coaxial.

The combined light is passed through homogenization optics 44, such as a fly-eye lens array, e.g. as described in US 6507434.

Two cylindrical lenses 46a, 46b, a further lens 46c, and the homogenization optics 44 together also widen the light beam along one direction, e.g. giving it an elongate cross-section, e.g. having a width-to-height ratio of 16:9, for better matching the typically available form factor of spatial light modulators.

A mirror 48 deflects the light into an assembly of two prisms 50a, 50b with a gap 52 between them.

The light beam passes prism 50a, gap 52, and prism 50b and arrives at spatial light modulator 24.

In the shown embodiment, spatial light modulator 24 is a DMD ("digital micro-mirror device") with a two-dimensional array of individually deflectable micro-mirrors. Control unit 32 is adapted to control the alignment of each micro-mirror, e.g. between a first and a second position.

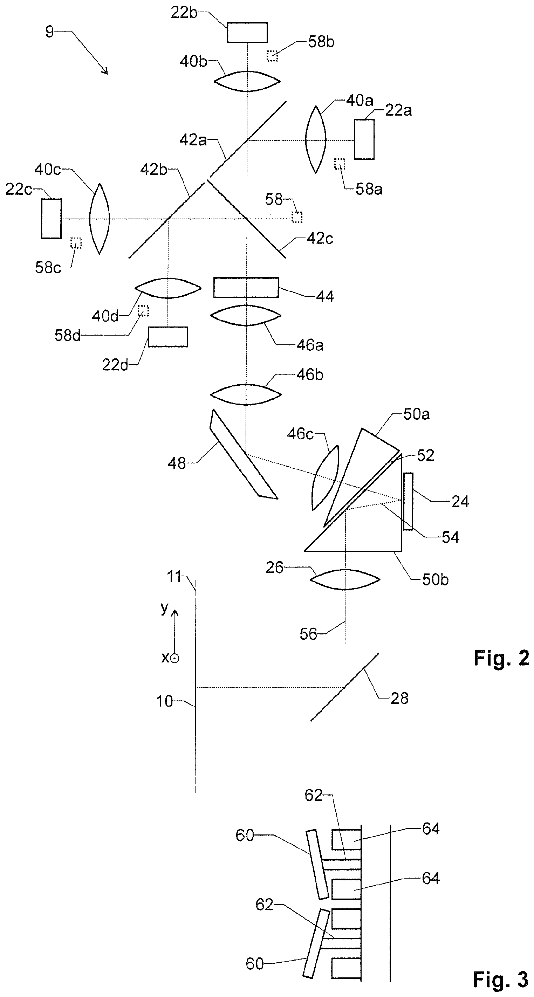

Fig. 3 shows, schematically, a sectional view of two micro-mirrors 60 of such a DMD. Each mirror 60 is held by an elastic support 62 and deflectable by means of electrodes 64. Devices of this type are known to the skilled person.

For the micro-mirrors 60 being in the first position, the light is reflected back into prism 50b along a direction denoted by 54 in Fig. 2. Light traveling along this direction 54 is subject to total internal reflection at the interface of second prism 50b to gap 52 and reflected into a direction denoted by 56 in Fig. 2.

For the micro-mirrors 60 being in the second position, the light is still reflected back into prism 50b, but along a different direction (not shown in Fig. 2), along which it does not fulfil the conditions for total internal reflection at the interface to gap 52. The small fraction still reflected at this interface will travel in a direction different from direction 56 and not be further processed by illumination imaging optics 26 described in the following.

Hence, control unit 32 is able to individually set each pixel (each micro-mirror 60) of spatial light modulator 24 into an on-state and an off-sate, thereby defining the contour and shape of the light field at target 10 (which is assumed to be located in the target plane 11 of the illumination device).

The light from the pixels of spatial light modulator 24 enters illumination imaging optics 26, which may include one or more lenses. From there, it may pass mirror 28 to arrive at target 10.

The illumination imaging optics images spatial light modulator 24 onto target 10, i.e. target 10 is at the target plane 11, which is the conjugate plane, in respect to illumination imaging optics 26, of the plane 62 of spatial light modulator 24.

*Operation*

The process of recording a series of frames by means of camera 16 is illustrated in Fig. 4.

As can be seen in the first line of Fig. 4, the camera runs through integration and processing phases, which may also at least partially overlap.

In each integration phase, the pixels of the camera integrate the light impinging on them. Then, the respective signal is locked, converted to a digital value, and read out by control unit 32 during a processing phase. This process repeats itself in repetitive frame cycles.

Advantageously, camera 16 is operated in free-running mode, i.e. it repetitively records frames without waiting for external trigger signals. This allows to operate camera 16 at a high frame rate.

Each integration phase is marked by a camera synch signal, as shown in the second line of Fig. 4. If camera 16 is operating in free-running mode, this is the signal on frame signal output 38a.

Depending on the specific hardware used, the signal "camera synch" may e.g. coincide with the beginning or the end of the integration phase.

Control unit 32 operates spatial light modulator 24 and light source 22 in synchronicity with the "camera synch" signal as shown in the third to fifth lines of Fig. 4.

In the shown embodiment, control unit 32 operates illumination device 9, during one frame cycle, in four operating phases I1, I2, I3, and I4. They are repeated in each frame cycle. The integration phase of the camera falls into operating phase I1, while the operating phases I2 - I4 are outside the integration phase.

In operating phase I1, spatial light modulator 24 is configured for the appropriate configuration (pattern) P1 of pixels. Advantageously, this configuration is completely set up before the integration phase starts (see fourth line of Fig. 4).

Only then, light source 22 is switched on (see fifth line of Fig. 4) to start a first light pulse L1.

In the shown example, configuration P1 is maintained over the whole integration phase. Also, the light source 22 remains switched on over the whole integration phase (light pulse L1).

When the integration phase has ended, light source 22 is switched off.

The integration phase may be shorter than first operating phase I1.

In operating phase I2, spatial light modulator 24 is brought into a second configuration (pattern) P2 while light source 22 remains switched off.

In operating phase I3, spatial light modulator 24 is brought into a third configuration (pattern) P3, and light source 22 is switched on again in order to generate a light pulse L2, which is switched off again before or at the end of operating phase I3.

In operating phase I4, spatial light modulator 24 is set to a fourth configuration (pattern) P4 and light source 22 remains switched off.

Then, the process repeats with phases I1 - I4 and with possible different configurations P1', P2', P3', P4'.

In one embodiment, the configurations are as follows:

- Configuration P1 corresponds to the illumination pattern that is desired during recording of the respective frame. It may remain unchanged over the whole integration phase. Alternatively, however, it may be changed during the integration phase, e.g. by switching individual pixels from their on-state to their off-state in order to individually decrease the illumination brightness of said pixels.
- Configuration P3 advantageously corresponds to configuration P1, i.e. it has the same pixels switched on and off as configuration P1. This generates two identical illumination patterns on the eye at twice the framerate, thereby reducing flicker as described above.
- Configurations P2 and P4 are advantageously the "opposite" of configurations P1 and P3, i.e. when a pixel is on in configuration P1 or P3, the same pixel is off in configuration P2 and P4, and vice versa. This is based on the understanding that the time to failure of DMD spatial light modulators can be increased by regularly bringing each mirror into both its positions for similar periods of time. The present embodiment uses the dark phases between the light pulses L1, L2 to do that.

Advantageously, the operating phases I1 - I4 have equal or similar lengths in order to compensate the strain in the supports 62 of the mirrors 60 of spatial light modulator 24. Advantageously, the longest one of the operating phases I1 - I4 is no more than 10 times, in particular no more than five times, in particular no more than 3 times, as long as the shortest one of the operating phases I1 - I4.

The scheme of Fig. 4 achieves two goals:

1. It balances the mirror deflection in spatial light modulator 24, thereby reducing aging of the modulator.
2. It allows to generate light pulses at the double framerate of camera 16, thereby reducing flicker.

In order to achieve goal 1, in more general terms, control unit 32 may be configured to bring, over one frame cycle (i.e. over one cycle of the integration and processing phase) of camera 16, i.e. for each frame, each micro-mirror 60 of spatial light modulator 24 into its first position during a time t1 and into its second, opposite position during a time t2, where t1 and t2 are comparable, i.e. where

$$0.1 < t1/t2 < 10, \qquad\qquad (1)$$

in particular wherein

$$0.2 < t1/t2 < 5, \qquad\qquad (2)$$

and in particular wherein

$$0.33 < t1/t2 < 3. \hspace{4cm} (3)$$

In order to achieve goal 2, in more general terms, control unit 32 may be configured to generate, over one frame cycle of camera 16, i.e. for each frame, at least two separate light pulses, wherein one of the light pulses falls into the integration phase of camera 16 while the other light pulse(s) falls outside the integration phase.

Advantageously, the light pulses L1, L2 have equal durations (in particular within 10%) and, for all light pulses, spatial light modulator 24 has the same configuration (pattern), and the light pulses are separated by dark phases of equal length (i.e. the dark phases have equal length, in particular within 10%). This further reduces flicker.

Advantageously, the repetition rate of the light pulses is at least 70 Hz.

If goal 1 is not required (e.g. because a spatial light modulator not sensitive to unbalanced pixel settings is used, e.g. an LCD modulator), the configuration of spatial light modulator 24 may be any pattern during operating phases I2 and I4.

If goal 2 is not required (e.g. because the frame rate of camera 16 is inherently high enough to avoid flicker effects), operating phases I3 and I4 may be omitted.

If goals 1 and 2 are both to be achieved, control unit 32 is advantageously adapted to bring spatial light modulator 24, between the light pulses L1, L2, into configurations P2 and P4 opposite to the configurations P1 and P3 during the light pulses. In this context, "opposite" first and second configurations means that that if, in the first configuration, a given pixel is in its first state during a time ta and its second state during a time tb, in the second configuration it will be (within an accuracy of 10% or better) in its first state during time tb and its second state during time ta.

If the process shown in Fig. 4 is to be synchronized to frame signal output 38a, the operating phases I1 - I4 should be synchronized to the integration phases of camera 16, such that each integration phase falls into a defined position of first operating phase I1.

To do so, control unit 32 is advantageously adapted to predict the time when camera 16 will record a next frame because, for example, it may need to configure spatial light modulator 24 before the integration phase of camera 16 starts, i.e. - in the embodiment of Fig. 4 - before the camera synch pulse arrives.

For this purpose, control unit 32 may e.g. use an estimate of the time tf between two consecutive frames.

This estimate for time tf can e.g. be a value stored in control unit 32 by the manufacturer. Alternatively, control unit 32 can measure it during operation, i.e. it can determine it from the times when camera 16 recorded previous frames.

Then, depending on the time when camera 16 recorded the last frame, i.e. depending on the time of the last camera synch pulse, the time of the next camera synch pulse, i.e. of the next integration phase, can be predicted.

This allows control unit 32 to prepare at least part of illumination device 9, such as spatial light modulator 34 and/or light source 22, before the predicted time of the next integration phase.

*Position Balancing of the micro-mirrors*

As mentioned above in reference to relation (1), (2), or (3), the positions of the micro-mirrors 60 are advantageously balanced, at least to some degree, in order to reduce mechanical strain.

This balancing takes place over no more than N consecutive frame cycles of the camera, i.e. it may not be fulfilled for each single frame cycle, but over the total times t1 and t2 over of a larger number N of frame cycles, with $N \leq 100$, may fulfill the relations (1), (2), or (3).

Advantageously, though, relation (1), (2), or (3) are fulfilled for a smaller number of cycles, such as $N \leq 10$ in order to reduce asymmetric strain further.

In a most advantageous embodiment, N = 1, i.e. balancing in the sense of relation (1), (2), or (3) takes place over each single frame cycle. This greatly simplifies the control of the device because it obviates the need to "plan" the mirror positions over two or more frame cycles.

In the period where the light source is operating and the camera is integrating, the position of each micro-mirror 60 is given by the amount of light to be generated for the respective pixel. Hence, balancing in order to fulfill relation (1), (2), or (3) advantageously takes place during the time when at least one of the following conditions is met:

- the camera is not integrating and/or
- the light source is not lit.

Advantageously, though, the balancing takes place when the light source is not lit, thereby reducing the risk of unwanted flicker in the patient's eye.

Note that t1 and t2 are typically different for at least some of the micro-mirrors, i.e. t1 and t2 are, in a most general case, dependent on the location i, j of the mirror in the modulator, i.e. t1(i,j) may differ from tl(i',j') if $i \neq i'$ and/or $j \neq j'$.

For example, one pixel may be kept dark while integrating the image, and another pixel may be kept bright while integrating the image. It may even be that a third pixel may be kept bright over a first part of the integration and switched to dark for a second part of the integration in order to achieve a pixel-wise brightness-modulation. In these cases, the times t1 and t2 may be different for both or all three types of pixels.

The present technique is particularly important if there is only a single light source or if there are several light sourced that are, however, switched on in an overlapping mater, i.e. if there is at least one time in the frame cycle (in particular during while the camera integrates an image, i.e. records a frame) where all the light sources are switched on. This is in contrast to RGB-projector-systems, where the color channels are projected consecutively, i.e. where only one color channel is active at a time - in such systems, balancing is achieved more easily.

Advantageously, imperfect balancing is used, which allows to carry out the balancing in a shorter time. Hence, advantageously, t1/t2 is larger than 0.6 or smaller than 0.4.

*Brightness and/or color monitoring*

Illumination device 9 may further comprise at least one light sensor for monitoring the brightness of the light source(s).

Such a sensor allows to monitor and control the brightness of the light sources 22a - 22d as their efficiency changes with temperature and depending on the age of the light sources.

In particular, control unit 32 can be adapted to monitor the brightness of the light sources for generating calibration parameters and to use these calibration parameters for controlling the light sources, e.g. by adjusting the currents to the light sources.

For example, and as shown in Fig. 2, there may be one such light sensor 58a, 58b, 58c, 58d attributed to each one light source 22a, 22b, 22c, 22d, which is positioned to receive part of the light from its light source and to generate a signal indicative of its brightness.

Advantageously, however, there may be a single light sensor 58 positioned to measure light from all of several light sources 22a - 22d. This can e.g. be achieved by a sensor 58 located at a position where the light from all light sources 22a - 22d is combined, e.g. by using the spurious reflection of dichroic beam splitter 42c, which is the last dichroic beam splitter. It is optimized to cast all light towards spatial light modulator 24. However, since it is, in reality, not an ideal dichroic beam splitter, a small fraction of the light from each light source is reflected out of the optical axis of the illumination device and can be used for monitoring the brightness by means of light sensor 58.

In this embodiment, in order to individually measure the brightness of the individual light sources, control unit 32 can be equipped to perform calibration measurements outside the integration phase of camera 16. Such a calibration measurements may comprise the steps of

- Placing spatial light modulator 24 into a non-transmitting configuration.
- Switching on exactly one of the light sources 22a - 22d and measuring a brightness signal for this light source by means of light sensor 58.

This scheme is, as mentioned, best performed when the device comprises a light sensor 58 positioned to measure light from all of several light sources. In particular, this light sensor is arranged to receive off-axial light from a last dichroic beam splitter used for combining the light of the light sources.

Such calibration phases are carried out for all light sources sequentially, either in the same frame cycle or in separate frame cycles of camera 16.

The calibration phases may e.g. be inserted during, immediately before or immediately after operating phases I2 or I4.

Optionally, the patterns in configurations I2 and I4 may be adapted to compensate for the time the mirrors are spending in their off-position during the calibration phases in order to maintain the condition of Eq. (1).

This kind of monitoring is particularly useful when the illumination device comprises several light sources 22a - 22d of different colors, such as red, green, and blue LEDs. In this case, the brightness of each one of them can be monitored, which allows to adjust the relative brightness values of the light sources in order to maintain a desired spectral composition (i.e. color tone, such as a desired color temperature of normally white light), advantageously in a control loop.

Hence, advantageously, the light sources 22a - 22d have different colors (i.e. their center wavelength differ by at least 20 nm). In that case, the method performed by the control unit of the device may comprise the step of using the calibration measurements for the light sources 22a - 22d in order to maintain a desired relative brightness between at least two of the light sources 22a - 22d when said at least two light sources are simultaneously switched on while recording a frame by means of the camera.

*Notes*

In the embodiments above, illumination device 9 comprises several individual light sources 22a - 22d, e.g. two, three, four or even more light sources. It may, however, also comprise only a single light source, such as a white LED.

In the first aspect of the invention, camera 16 generates the master signal for triggering illumination device 9, i.e. the signal "camera synch" in Fig. 3 is generated by camera 16. In other implementations of the invention, however, camera 16 may also operate in triggered mode, i.e. it integrates a frame upon receiving an external trigger. In that case, control unit 32 may e.g. generate the trigger signal, triggering the recording of a frame by means of camera 16, i.e. the signal "camera synch" in Fig. 4 may not be generated by camera 16 but by control unit 32.

While there are shown and described presently preferred embodiments of the invention, it is to be distinctly understood that the invention is not limited thereto but may be otherwise variously embodied and practiced within the scope of the following claims.

**Claims**

1. An ophthalmologic microscope comprising

    i) an illumination device (9) configured to generate illumination pulses (L1, L2) for illuminating an eye to be examined and having

        a) at least one light source (22, 22a - 22d),
        b) a spatial light modulator (24), and
        c) illumination imaging optics (26) structured to project an image of the spatial light modulator onto the eye,

    ii) a microscope device (8) adapted to record an image of the eye and having

        a) microscope optics (14, 15) configured to generate an image of the eye and
        b) at least one electronic camera (16) adapted to record the image of the eye, and

    iii) a control unit (32),

        wherein said spatial light modulator (24) is an electronically controlled spatial light modulator (24) comprising a two-dimensional array of individually controllable pixels,
        **characterised in that**
        said camera (16) has a frame signal output (38a) carrying a signal indicative of times when the camera (16) records a frame and
        wherein said control unit (32) is structured to synchronize said illumination device (9) to said frame signal output (38a),
        and wherein said control unit (32) is structured to synchronize a state of said pixels to said frame signal output (38a).

2. The microscope claim 1
    wherein said control unit (32) is structured to generate at least two illumination pulses (L1, L2) for each frame recorded by said camera (16).

3. The microscope of any of the preceding claims wherein said camera (16) is configured to operate in free-running mode.

4. The microscope of any of the preceding claims wherein said spatial light modulator (24) comprises a two-dimensional array of micro-mirrors (60) individually deflectable into a first and a second position.

5. The microscope of claim 4 wherein said control unit (32) is structured to bring, for a group of no more than N consecutive frame cycles of said camera (16), each micro-mirror (60) of said spatial light modulator (24) into the first position during a time t1 and into the second position during a time t2, wherein

$$0.1 < t1/t2 < 10,$$

in particular wherein

$$0.2 < t1/t2 < 5,$$

and in particular wherein

$$0.33 < t1/t2 < 3,$$

wherein $N \leq 100$, in particular $N \leq 10$, in particular $N = 1$.

6. The microscope of any of the preceding claims wherein said control unit (32) is structured to pulse said light source (22, 22a - 22d), in particular in a manner synchronized to said frame signal output (38a).

7. The microscope of claim 6, wherein the control unit (32) is structured to

   - bringing the pixels into a given configuration during a dark phase prior to a given light pulse, and
   - starting the light pulse only when the pixels are in the given configuration.

8. The microscope of any of the claims 6 or 7 wherein said control unit (32) is structured to generate, for each frame recorded by said camera (16), at least two separate light pulses (L1, L2), wherein one (L1) of the light pulses falls into the integration phase of the camera (16) while the other (L2) light pulse(s) fall outside the integration phase.

9. The microscope of claim 8, wherein

   the light pulses (L1, L2) have equal durations,
   for all light pulses (L1, L2), the spatial light modulator (24) has the same configurations (P1, P3), and
   the light pulses are separated by dark phases of equal length.

10. The microscope of claim 9 wherein said control unit (32) is adapted to bring, between the light pulses (L1, L2) the spatial light modulator (24) into configurations (P2, P4) opposite to the configurations (P1, P3) during the light pulses (L1, L2).

11. The microscope of any of the preceding claims wherein said control unit (32) is adapted to

   - predicting a time when said camera (16) will record a next frame depending on the time when the camera (16) recorded at least one previous frame, in particular depending on the times when the camera (16) recorded several previous frames, and
   - preparing at least part of said illumination device (9) before the predicted time for recording the next frame.

12. The microscope of any of the preceding claims further comprising at least one light sensor (58a - 58d, 58) adapted to measure a light intensity between said light source (22, 22a - 22d) and said spatial light modulator (24), wherein said control unit (32) is structured to

   - bringing said light modulator (24) into a non-transmitting mode, and
   - while said light modulator (24) is in said non-transmitting mode, pulsing said at least one light source (22, 22a - 22d) for measuring a brightness of said light source (22, 22a - 22d).

13. The microscope of any of the preceding claims further comprising at least one light sensor (58a - 58d, 58) for monitoring a brightness of the light source (22, 22a - 22d), wherein said control unit (32) is adapted to monitor the brightness of the light source (22, 22a - 22d) by means of said light sensor (58a - 58d, 58) for generating calibration parameters and to use the calibration parameters for controlling the at least one light source (22, 22a - 22d).

14. The microscope of claim 13 comprising several light sources (22a - 22d), wherein said control unit (32) is adapted to carry out calibration measurements outside an integration phase of said camera (16), wherein said calibration measurements comprise the steps of

   - placing said spatial light modulator (24) into a non-transmitting configuration and
   - switching on exactly one of the light sources (22a - 22d) and measuring a brightness signal by means of light sensor (58).

15. A method for operating an ophthalmologic microscope, in particular the ophthalmologic microscope of any of the preceding claims, wherein said device comprises

    i) an illumination device (9) for illuminating an eye to be examined having

        a) at least one light source (22, 22a - 22d),
        b) a spatial light modulator (24), and
        c) illumination imaging optics (26) structured to project an image of the spatial light modulator onto the eye,

    ii) a microscope device (8) adapted to record an image of the eye and having

        a) microscope optics (14, 15) configured to generate an image of the eye and
        b) at least one electronic camera (16) adapted to record the image of the eye, and

    iii) a control unit (32),

    wherein said camera (16) has a frame signal output (38a) carrying a signal indicative of times when the camera (16) records a frame and
    wherein said method comprises the steps of sequentially recording frames by means of said camera (16) and generating, by means of the illumination device (9), light pulses synchronized to said frame signal output (38a),
    wherein said spatial light modulator (24) is an electronically controlled spatial light modulator (24) comprising a two-dimensional array, wherein said pixels are controlled individually, and wherein said method comprises the step of controlling the pixels in a manner synchronized to said frame signal output (38a).

16. The method of claim 15 further comprising the step of
generating, by means of said illumination device (9), at least two illumination pulses (L1, L2) for each frame recorded by said camera (16).

17. The method of any of the claims 15 or 16 wherein said camera (16) is run in free-running mode.

18. The method of any of the claims 16 or 17, wherein said spatial light modulator (24) comprises a two-dimensional array of micro-mirrors (60), wherein said micro-mirrors (60) are individually deflected into a first and a second position.

19. The method of claim 18 comprising, for each frame recorded by said camera (16), the step of bringing each micro-mirror (60) of said spatial light modulator (24) into the first position during a time t1 and into the second position during a time t2, wherein t1 and t2 are equal.

20. The method of any of the claims 15 to 19 comprising the step of pulsing said light source (22, 22a - 22d), in particular in a manner synchronized to said frame signal output (38a).

21. The method of claim 20 comprising the steps of

    - bringing the pixels into a given configuration during a dark phase prior to a given light pulse, and
    - starting the light pulse only when the pixels are in the given configuration.

22. The method of any of the claims 20 or 21 comprising the step of generating, for each frame recorded by said camera (16), at least two separate light pulses (L1, L2), wherein one (L1) of the light pulses falls into the integration phase of the camera (16) while the other (L2) light pulse(s) fall outside the integration phase.

23. The method of claim 22 wherein

    the light pulses (L1, L2) have equal durations,
    for all light pulses (L1, L2), the spatial light modulator (24) has the same configurations (P1, P3), and
    the light pulses are separated by dark phases of equal length.

24. The method of claim 23 comprising the steps of bringing, between the light pulses (L1, L2), the spatial light modulator (24) into configurations (P2, P4) opposite to the configurations (P1, P3) during the light pulses (L1, L2).

25. The method of any of the claims 15 to 24 comprising the steps of

predicting a time when said camera (16) will record a next frame depending on the time when the camera (16) recorded at least one previous frame, in particular depending on the times when the camera (16) recorded several previous frames, and
preparing at least part of said illumination device (9) before the predicted time for recording the next frame.

26. The method of any of the claims 15 to 25, wherein said device further comprises at least one light sensor adapted to measure a light intensity between said light source (22, 22a - 22d) and said spatial light modulator (24), wherein said method comprises the steps of

- bringing said light modulator into a non-transmitting mode, and
- while said light modulator is in said non-transmitting mode, pulsing said at least one light source (22, 22a - 22d) for measuring a brightness of said light source (22).

27. The method of any of the claims 15 to 26, wherein said microscope comprises at least one light sensor (58a - 58d, 58) and comprising the steps of

monitoring a brightness of the light source (22, 22a - 22d) by means of said light sensor (58a - 58d, 58) and generating calibration parameters and
using the calibration parameters for controlling the light source (22, 22a - 22d).

28. The method of claim 27, wherein said microscope comprises several light sources (22a - 22d), wherein said method comprises the step of calibration measurements outside an integration phase of said camera (16), wherein said calibration measurements comprise the steps of

- placing said spatial light modulator (24) into a non-transmitting configuration and
- switching on exactly one of the light sources (22a - 22d) and measuring a brightness signal by means of light sensor (58),

and in particular wherein said light sources (22a - 22d) have different colors and wherein said method further comprises the step of using the calibration measurements for the light sources (22a - 22d) in order to maintain a desired relative brightness between at least two of the light sources (22a - 22d) when said two light sources are simultaneously switched on while recording a frame.

**Patentansprüche**

1. Ophthalmologisches Mikroskop, umfassend

i) eine Beleuchtungseinrichtung (9), die so konfiguriert ist, dass sie Lichtpulse (L1, L2) zum Beleuchten des zu untersuchenden Auges erzeugt, und die Folgendes aufweist

a) mindestens eine Lichtquelle (22, 22a - 22d),
b) einen räumlichen Lichtmodulator (24) und
c) eine Beleuchtungsabbildungsoptik (26), die so strukturiert ist, dass sie ein Bild des räumlichen Lichtmodulators auf das Auge projiziert,

ii) eine Mikroskopvorrichtung (8), die zum Aufzeichnen eines Bildes des Auges ausgstaltet ist und Folgendes aufweist

a) eine Mikroskopoptik (14, 15), die zum Erzeugen eines Bildes des Auges konfiguriert ist, und
b) mindestens eine elektronische Kamera (16), die dazu ausgestaltet ist, das Bild des Auges aufzuzeichnen, und

iii) eine Steuereinheit (32),

wobei der räumliche Lichtmodulator (24) ein elektronisch gesteuerter räumlicher Lichtmodulator (24) ist, der eine zweidimensionale Anordnung einzeln steuerbarer Pixel umfasst **dadurch gekennzeichnet, dass** die Kamera (16) einen Bildsignalausgang (38a) aufweist, der ein Signal überträgt, das angibt, wann die Kamera (16) ein Bild aufzeichnet, und wobei die Steuereinheit (32) derart strukturiert ist, dass sie die Beleuchtungsvorrichtung (9) mit dem Bildsignalausgang (38a) synchronisiert, und wobei die Steuereinheit (32) derart strukturiert ist, dass sie einen Zustand der Pixel mit dem Bildsignalausgang (38a) synchronisiert.

2. Mikroskop nach Anspruch 1 wobei die Steuereinheit (32) derart strukturiert ist, dass sie mindestens zwei Lichtpulse (L1, L2) für jedes von der Kamera (16) aufgezeichnete Bild erzeugt.

3. Mikroskop nach einem der vorstehenden Ansprüche, wobei die Kamera (16) derart konfiguriert ist, dass sie im Freilaufmodus arbeitet.

4. Mikroskop nach einem der vorstehenden Ansprüche, wobei der räumliche Lichtmodulator (24) eine zweidimensionale Anordnung von Mikrospiegeln (60) umfasst, die einzeln in eine erste und eine zweite Position abgelenkt werden können.

5. Mikroskop nach Anspruch 4, wobei die Steuereinheit (32) derart strukturiert ist, dass sie für eine Gruppe von nicht mehr als N aufeinanderfolgenden Bildzyklen der Kamera (16) jeden Mikrospiegel (60) des räumlichen Lichtmodulators (24) während einer Zeit t1 in die erste Position und während einer Zeit t2 in die zweite Position bringt, wobei

$$0.1 < t1/t2 < 10,$$

insbesondere wobei

$$0.2 < t1/t2 < 5,$$

und insbesondere wobei

$$0.33 < t1/t2 < 3,$$

wobei $N \leq 100$, insbesondere $N \leq 10$, insbesondere $N = 1$.

6. Mikroskop nach einem der vorstehenden Ansprüche, wobei die Steuereinheit (32) derart strukturiert ist, dass sie die Lichtquelle (22, 22a - 22d) pulsiert, insbesondere in einer mit dem Bildsignalausgang (38a) synchronisierten Weise.

7. Mikroskop nach Anspruch 6, wobei die Steuereinheit (32) derart strukturiert ist, dass sie

- die Pixel während einer Dunkelphase vor einem gegebenen Lichtimpuls in eine bestimmte Konfiguration bringt und
- den Lichtimpuls erst dann startet, wenn sich die Pixel in der vorgegebenen Konfiguration befinden.

8. Mikroskop nach einem der Ansprüche 6 oder 7, wobei die Steuereinheit (32) derart strukturiert ist, dass sie für jedes von der Kamera (16) aufgezeichnete Bild mindestens zwei separate Lichtpulse (L1, L2) erzeugt, wobei einer (L1) der Lichtpulse in die Integrationsphase der Kamera (16) fällt, während der andere (L2) Lichtimpuls außerhalb der Integrationsphase liegt.

9. Mikroskop nach Anspruch 8, wobei

die Lichtpulse (L1, L2) gleiche Dauer haben,

für alle Lichtpulse (L1, L2) der räumliche Lichtmodulator (24) die gleichen Konfigurationen (P1, P3) aufweist und die Lichtimpulse durch Dunkelphasen gleicher Länge voneinander getrennt sind.

10. Mikroskop nach Anspruch 9, wobei die Steuereinheit (32) so ausgestaltet ist, dass sie den räumlichen Lichtmodulator (24) zwischen den Lichtpulse (L1, L2) in Konfigurationen (P2, P4) bringt, die den Konfigurationen (P1, P3) während der Lichtpulse (L1, L2) entgegengesetzt sind.

11. Mikroskop nach einem der vorstehenden Ansprüche, wobei die Steuereinheit (32) dazu ausgestaltet ist,

- einen Zeitpunkt vorherzusagen, zu dem die Kamera (16) ein nächstes Bild aufzeichnet, abhängig von dem Zeitpunkt, zu dem die Kamera (16) mindestens ein vorheriges Bild aufgezeichnet hat, insbesondere abhängig von den Zeitpunkten, zu denen die Kamera (16) mehrere vorherige Bilder aufgezeichnet hat, und
- mindestens einen Teil der Beleuchtungseinrichtung (9) vor dem vorhergesagten Zeitpunkt für die Aufnahme des nächsten Bildes vorbereitet.

12. Mikroskop nach einem der vorstehenden Ansprüche, das ferner mindestens einen Lichtsensor (58a - 58d, 58) aufweist, der dazu ausgelegt ist, eine Lichtintensität zwischen der Lichtquelle (22, 22a - 22d) und dem räumlichen Lichtmodulator (24) zu messen, wobei die Steuereinheit (32) derart strukturiert ist, dass sie

- den Lichtmodulator (24) in einen nicht transmittierenden Modus versetzt und
- während sich der Lichtmodulator (24) in dem nicht transmittierenden Modus befindet, die mindestens eine Lichtquelle (22, 22a - 22d) pulsiert, um eine Helligkeit der Lichtquelle (22, 22a - 22d) zu messen.

13. Mikroskop nach einem der vorstehenden Ansprüche, das ferner mindestens einen Lichtsensor (58a - 58d, 58) zum Überwachen einer Helligkeit der Lichtquelle (22, 22a - 22d) aufweist, wobei die Steuereinheit (32) dazu ausgestaltet ist, die Helligkeit der Lichtquelle (22, 22a - 22d) mittels des Lichtsensors (58a - 58d, 58) zu überwachen, um Kalibrierungsparameter zu erzeugen, und die Kalibrierungsparameter zur Steuerung der mindestens einen Lichtquelle (22, 22a - 22d) zu verwenden.

14. Mikroskop nach Anspruch 13, umfassend mehrere Lichtquellen (22a - 22d), wobei die Steuereinheit (32) dazu ausgelegt ist, Kalibrierungsmessungen ausserhalb einer Integrationsphase der Kamera (16) durchzuführen, wobei die Kalibrierungsmessungen die folgenden Schritte umfassen

- Versetzen des räumlichen Lichtmodulators (24) in eine nicht transmittierende Konfiguration und
- genau eine der Lichtquellen (22a - 22d) einschalten und ein Helligkeitssignal mittels eines Lichtsensors (58) messen.

15. Verfahren zum Betreiben eines ophthalmologischen Mikroskops, insbesondere des ophthalmologischen Mikroskops nach einem der vorstehenden Ansprüche, wobei die Vorrichtung umfasst

i) eine Beleuchtungseinrichtung (9) zum Beleuchten eines zu untersuchenden Auges mit

a) mindestens einer Lichtquelle (22, 22a - 22d),
b) einem räumlichen Lichtmodulator (24) und
c) einer Beleuchtungsabbildungsoptik (26), die derart strukturiert ist, dass sie ein Bild des räumlichen Lichtmodulators auf das Auge projiziert,

ii) eine Mikroskopvorrichtung (8), die zum Aufzeichnen eines Bildes des Auges ausgestaltet ist und Folgendes aufweist

a) eine Mikroskopoptik (14, 15), die derart konfiguriert ist, dass sie ein Bild des Auges erzeugt, und
b) mindestens eine elektronische Kamera (16), die dazu ausgestaltet ist, das Bild des Auges aufzuzeichnen, und

iii) eine Steuereinheit (32),

wobei die Kamera (16) einen Bildsignalausgang (38a) aufweist, der ein Signal überträgt, das angibt, wann die Kamera (16) ein Bild aufzeichnet, und

wobei das Verfahren die folgenden Schritte umfasst
sequentielles Aufzeichnen von Bildern mittels der Kamera (16) und
Erzeugen von Lichtimpulsen mittels der Beleuchtungsvorrichtung (9), die mit dem Bildsignalausgang (38a) synchronisiert sind,
wobei der räumliche Lichtmodulator (24) ein elektronisch gesteuerter räumlicher Lichtmodulator (24) ist, der eine zweidimensionale Anordnung umfasst, wobei die Pixel einzeln gesteuert werden, und wobei das Verfahren den Schritt aufweist, die Pixel in einer mit dem Bildsignalausgang (38a) synchronisierten Weise zu steuern.

16. Verfahren nach Anspruch 15, das ferner den Schritt umfasst Erzeugen von mindestens zwei Lichtpulse (L1, L2) für jedes von der Kamera (16) aufgezeichnete Bild mittels der Beleuchtungsvorrichtung (9).

17. Verfahren nach einem der Ansprüche 15 oder 16, wobei die Kamera (16) im Freilaufmodus betrieben wird.

18. Verfahren nach einem der Ansprüche 16 oder 17, wobei der räumliche Lichtmodulator (24) eine zweidimensionale Anordnung von Mikrospiegeln (60) umfasst, wobei die Mikrospiegel (60) einzeln in eine erste und eine zweite Position abgelenkt werden.

19. Verfahren nach Anspruch 18, das für jedes von der Kamera (16) aufgezeichnete Bild den Schritt umfasst, jeden Mikrospiegel (60) des räumlichen Lichtmodulators (24) während einer Zeit t1 in die erste Position und während einer Zeit t2 in die zweite Position zu bringen, wobei t1 und t2 gleich sind.

20. Verfahren nach einem der Ansprüche 15 bis 19, das den Schritt des Pulsierens der Lichtquelle (22, 22a - 22d) umfasst, insbesondere in einer Weise, die mit der Bildsignalausgabe (38a) synchronisiert ist.

21. Verfahren nach Anspruch 20, umfassend die Schritte

- die Pixel während einer Dunkelphase vor einem bestimmten Lichtimpuls in eine bestimmte Konfiguration bringen, und
- starten des Lichtimpulses nur dann, wenn sich die Pixel in der vorgegebenen Konfiguration befinden.

22. Verfahren nach einem der Ansprüche 20 oder 21, umfassend den Schritt des Erzeugens von mindestens zwei separaten Lichtpulsen (L1, L2) für jedes von der Kamera (16) aufgezeichnete Bild, wobei einer (L1) der Lichtpulse in die Integrationsphase der Kamera (16) fällt, während der andere (L2) Lichtpuls ausserhalb der Integrationsphase fällt.

23. Verfahren nach Anspruch 22, wobei

die Lichtpulse (L1, L2) gleiche Dauer haben,
für alle Lichtpulse (L1, L2) der räumliche Lichtmodulator (24) die gleichen Konfigurationen (P1, P3) aufweist und
die Lichtimpulse durch Dunkelphasen gleicher Länge voneinander getrennt sind.

24. Verfahren nach Anspruch 23, das die Schritte umfasst, zwischen den Lichtpulsen (L1, L2) den räumlichen Lichtmodulator (24) in Konfigurationen (P2, P4) zu bringen, die den Konfigurationen (P1, P3) während der Lichtpulse (L1, L2) entgegengesetzt sind.

25. Verfahren nach einem der Ansprüche 15 bis 24, das die folgenden Schritte umfasst

Vorhersagen eines Zeitpunkts, zu dem die Kamera (16) ein nächstes Bild aufzeichnet, in Abhängigkeit von dem Zeitpunkt, zu dem die Kamera (16) mindestens ein vorheriges Bild aufgezeichnet hat, insbesondere in Abhängigkeit von den Zeitpunkten, zu denen die Kamera (16) mehrere vorherige Bilder aufgezeichnet hat, und
Vorbereiten mindestens eines Teils der Beleuchtungseinrichtung (9) vor dem für die Aufnahme des nächsten Bildes vorhergesagten Zeitpunkt.

26. Verfahren nach einem der Ansprüche 15 bis 25, wobei die Vorrichtung ferner mindestens einen Lichtsensor umfasst, der dazu ausgelegt ist, eine Lichtintensität zwischen der Lichtquelle (22, 22a - 22d) und dem räumlichen Lichtmodulator (24) zu messen,
wobei das Verfahren die folgenden Schritte umfasst:

- Versetzen des Lichtmodulators in einen nicht-transmittierenden Modus und
- während sich der Lichtmodulator in dem nicht- transmittierenden Modus befindet, Pulsieren der mindestens einen Lichtquelle (22, 22a - 22d), um eine Helligkeit der Lichtquelle (22) zu messen.

27. Verfahren nach einem der Ansprüche 15 bis 26, wobei das Mikroskop mindestens einen Lichtsensor (58a - 58d, 58) umfasst, mit den folgenden Schritten:

Überwachen der Helligkeit der Lichtquelle (22, 22a - 22d) mittels des Lichtsensors (58a - 58d, 58) und Erzeugen von Kalibrierungsparametern und
Verwenden der Kalibrierungsparameter zum Steuern der Lichtquelle (22, 22a - 22d).

28. Verfahren nach Anspruch 27, wobei das Mikroskop mehrere Lichtquellen (22a - 22d) umfasst, wobei das Verfahren den Schritt von Kalibrierungsmessungen ausserhalb einer Integrationsphase der Kamera (16) umfasst, wobei die Kalibrierungsmessungen die folgenden Schritte umfassen

- Anordnen des räumlichen Lichtmodulators (24) in einer nicht durchlässigen Konfiguration und
- Einschalten genau einer der Lichtquellen (22a - 22d) und Messen eines Helligkeitssignals mittels des Lichtsensors (58),

und wobei insbesondere die Lichtquellen (22a - 22d) unterschiedliche Farben aufweisen und wobei das Verfahren ferner den Schritt umfasst des Verwendens der Kalibrierungsmessungen für die Lichtquellen (22a - 22d), um eine gewünschte relative Helligkeit zwischen mindestens zwei der Lichtquellen (22a - 22d) aufrechtzuerhalten, wenn die beiden Lichtquellen gleichzeitig eingeschaltet sind, während ein Bild aufgezeichnet wird.

## Revendications

1. Un microscope ophtalmologique comprenant

i) un dispositif d'éclairage (9) configuré pour générer des impulsions d'éclairage (L1, L2) afin d'éclairer un œil à examiner et comportant

a) au moins une source de lumière (22, 22a - 22d),
b) un modulateur spatial de lumière (24), et
c) une optique d'imagerie d'éclairage (26) structurée pour projeter une image du modulateur spatial de lumière sur l'œil,

ii) un dispositif de microscope (8) adapté pour enregistrer une image de l'œil et comportant

a) une optique de microscope (14, 15) configurée pour générer une image de l'œil et
b) au moins une caméra électronique (16), adaptée pour enregistrer l'image de l'œil et

iii) une unité de commande (32),

dans lequel ledit modulateur spatial de lumière (24) est un modulateur spatial de lumière à commande électronique (24) comprenant un réseau bidimensionnel de pixels contrôlables individuellement **caractérisé en ce que**
ladite caméra (16) comporte une sortie de signal d'image (38a) transportant un signal indiquant les moments où la caméra (16) enregistre une image et
dans lequel ladite unité de commande (32) est structurée pour synchroniser ledit dispositif d'éclairage (9) avec ladite sortie de signal d'image (38a),
et dans lequel ladite unité de commande (32) est structurée pour synchroniser un état desdits pixels avec ladite sortie de signal d'image (38a).

2. Le microscope selon la revendication 1
dans lequel ladite unité de commande (32) est structurée pour générer au moins deux impulsions d'éclairage (L1, L2) pour chaque image enregistrée par ladite caméra (16).

3. Le microscope selon l'une quelconque des revendications précédentes, dans lequel ladite caméra (16) est configurée pour fonctionner en mode libre.

4. Le microscope selon l'une quelconque des revendications précédentes, dans lequel ledit modulateur spatial de lumière (24) comprend un réseau bidimensionnel de micro-miroirs (60) pouvant être déviés individuellement dans une première et une deuxième position.

5. Le microscope selon la revendication 4, dans lequel ladite unité de commande (32) est structurée pour amener, pour un groupe de pas plus de N cycles d'images consécutifs de ladite caméra (16), chaque micro-miroir (60) dudit modulateur spatial de lumière (24) dans la première position pendant un temps t1 et dans la deuxième position pendant un temps t2, où

$$0,1 < t1/t2 < 10,$$

en particulier dans lequel

$$0,2 < t1/t2 < 5,$$

et en particulier dans lequel

$$0,33 < t1/t2 < 3,$$

dans lequel $N \leq 100$, en particulier $N \leq 10$, en particulier $N = 1$.

6. Le microscope selon l'une quelconque des revendications précédentes, dans lequel ladite unité de commande (32) est structurée pour pulser ladite source de lumière (22, 22a - 22d), en particulier d'une manière synchronisée avec ladite sortie de signal d'image (38a).

7. Le microscope selon la revendication 6, dans lequel l'unité de commande (32) est structurée pour

- amener les pixels dans une configuration donnée pendant une phase sombre précédant une impulsion de lumière donnée, et
- démarrer l'impulsion de lumière uniquement lorsque les pixels sont dans la configuration donnée.

8. Le microscope selon l'une quelconque des revendications 6 ou 7, dans lequel ladite unité de commande (32) est structurée pour générer, pour chaque trame enregistrée par ladite caméra (16), au moins deux impulsions de lumière distinctes (L1, L2), dans lequel l'une (L1) des impulsions de lumière tombe dans la phase d'intégration de la caméra (16) tandis que l'autre (L2) impulsion de lumière tombe en dehors de la phase d'intégration.

9. Le microscope selon la revendication 8, dans lequel

les impulsions de lumière (L1, L2) ont des durées égales,
pour toutes les impulsions de lumière (L1, L2), le modulateur spatial de lumière (24) a les mêmes configurations (P1, P3), et
les impulsions de lumière sont séparées par des phases sombres de longueur égale.

10. Le microscope selon la revendication 9, dans lequel ladite unité de commande (32) est adaptée pour amener, entre les impulsions de lumière (L1, L2), le modulateur spatial de lumière (24) dans des configurations (P2, P4) opposées aux configurations (P1, P3) pendant les impulsions de lumière (L1, L2).

11. Le microscope selon l'une quelconque des revendications précédentes, dans lequel ladite unité de commande (32) est adaptée pour

- prédire un moment où ladite caméra (16) enregistrera une image suivante en fonction du moment où la caméra (16) a enregistré au moins une image précédente, en particulier en fonction des moments où la caméra (16) a enregistré plusieurs images précédentes, et
- préparer au moins une partie dudit dispositif d'éclairage (9) avant le moment prédit pour enregistrer l'image suivante.

**12.** Le microscope selon l'une quelconque des revendications précédentes, comprenant en outre au moins un capteur de lumière (58a - 58d, 58) adapté pour mesurer une intensité de lumière entre ladite source de lumière (22, 22a - 22d) et ledit modulateur spatial de lumière (24), dans lequel ladite unité de commande (32) est structurée pour

- mettre ledit modulateur de lumière (24) dans un mode non transmissif, et
- tandis que ledit modulateur de lumière (24) est dans ledit mode non transmissif, faire pulser ladite au moins une source de lumière (22, 22a - 22d) pour mesurer une luminosité de ladite source de lumière (22, 22a - 22d).

**13.** Le microscope selon l'une quelconque des revendications précédentes, comprenant en outre au moins un capteur de lumière (58a - 58d, 58) pour surveiller la luminosité de la source de lumière (22, 22a - 22d), dans lequel ladite unité de commande (32) est adaptée pour surveiller la luminosité de la source de lumière (22, 22a - 22d) au moyen dudit capteur de lumière (58a - 58d, 58) afin de générer des paramètres d'étalonnage et d'utiliser lesdits paramètres d'étalonnage pour commander ladite au moins une source de lumière (22, 22a - 22d).

**14.** Le microscope selon la revendication 13, comprenant plusieurs sources de lumière (22a à 22d), dans lequel ladite unité de commande (32) est adaptée pour effectuer des mesures d'étalonnage en dehors d'une phase d'intégration de ladite caméra (16), dans lequel lesdites mesures d'étalonnage comprennent les étapes consistant à

- placer ledit modulateur spatial de lumière (24) dans une configuration non transmissive et
- allumer exactement une des sources de lumière (22a à 22d) et mesurer un signal de luminosité à l'aide d'un capteur de lumière (58).

**15.** Un procédé pour faire fonctionner un microscope ophtalmologique, en particulier le microscope ophtalmologique selon l'une quelconque des revendications précédentes, dans lequel ledit dispositif comprend

i) un dispositif d'éclairage (9) pour éclairer un œil à examiner, comportant

a) au moins une source de lumière (22, 22a à 22d),
b) un modulateur spatial de lumière (24), et
c) une optique d'imagerie d'éclairage (26) structurée pour projeter une image du modulateur spatial de lumière sur l'œil,

ii) un dispositif de microscope (8) adapté pour enregistrer une image de l'œil et comportant

a) une optique de microscope (14, 15) configurée pour générer une image de l'œil et
b) au moins une caméra électronique (16) adaptée pour enregistrer l'image de l'œil, et

iii) une unité de commande (32),

dans lequel ladite caméra (16) comporte une sortie de signal d'image (38a) transportant un signal indicatif des moments où la caméra (16) enregistre une image, et
dans lequel ledit procédé comprend les étapes consistant à
enregistrer séquentiellement des images au moyen de ladite caméra (16) et
générer, au moyen du dispositif d'éclairage (9), des impulsions de lumières synchronisées avec ladite sortie de signal d'image (38a),
dans lequel ledit modulateur spatial de lumière (24) est un modulateur spatial de lumière à commande électronique (24) comprenant un réseau bidimensionnel, dans lequel lesdits pixels sont commandés individuellement, et dans lequel ledit procédé comprend l'étape consistant à commander les pixels d'une manière synchronisée avec ladite sortie de signal d'image (38a).

**16.** Le procédé selon la revendication 15, comprenant en outre l'étape consistant à
générer, au moyen dudit dispositif d'éclairage (9), au moins deux impulsions d'éclairage (L1, L2) pour chaque image enregistrée par ladite caméra (16).

**17.** Le procédé selon l'une quelconque des revendications 15 ou 16, dans lequel ladite caméra (16) fonctionne en mode libre.

**18.** Le procédé selon l'une quelconque des revendications 16 ou 17, dans lequel ledit modulateur spatial de lumière (24) comprend un réseau bidimensionnel de micro-miroirs (60), dans lequel lesdits micro-miroirs (60) sont déviés individuellement dans une première et une deuxième position.

**19.** Le procédé selon la revendication 18, comprenant, pour chaque image enregistrée par ladite caméra (16), l'étape consistant à amener chaque micro-miroir (60) dudit modulateur spatial de lumière (24) dans la première position pendant un temps t1 et dans la deuxième position pendant un temps t2, où t1 et t2 sont égaux.

**20.** Le procédé selon l'une quelconque des revendications 15 à 19, comprenant l'étape consistant à pulser ladite source de lumière (22, 22a à 22d), en particulier de manière synchronisée avec ladite sortie de signal d'image (38a).

**21.** Le procédé selon la revendication 20, comprenant les étapes consistant à

- amener les pixels dans une configuration donnée pendant une phase sombre précédant une impulsion de lumière donnée, et
- ne démarrer l'impulsion de lumière que lorsque les pixels sont dans la configuration donnée.

**22.** Le procédé selon l'une quelconque des revendications 20 ou 21, comprenant l'étape consistant à générer, pour chaque trame enregistrée par ladite caméra (16), au moins deux impulsions de lumière distinctes (L1, L2), dans lequel l'une (L1) des impulsions de lumière tombe dans la phase d'intégration de la caméra (16) tandis que l'autre (L2) impulsion de lumière tombe en dehors de la phase d'intégration.

**23.** Le procédé selon la revendication 22, dans lequel

les impulsions de lumière (L1, L2) ont des durées égales,
pour toutes les impulsions de lumière (L1, L2), le modulateur spatial de lumière (24) a les mêmes configurations (P1, P3), et
les impulsions de lumière sont séparées par des phases sombres de longueur égale.

**24.** Le procédé selon la revendication 23, comprenant les étapes consistant à amener, entre les impulsions de lumière (L1, L2), le modulateur spatial de lumière (24) dans des configurations (P2, P4) opposées aux configurations (P1, P3) pendant les impulsions de lumière (L1, L2).

**25.** Le procédé selon l'une quelconque des revendications 15 à 24, comprenant les étapes consistant à

prédire un moment où ladite caméra (16) enregistrera une image suivante en fonction du moment où la caméra (16) a enregistré au moins une image précédente, en particulier en fonction des moments où la caméra (16) a enregistré plusieurs images précédentes, et
préparer au moins une partie dudit dispositif d'éclairage (9) avant le moment prédit pour enregistrer l'image suivante.

**26.** Le procédé selon l'une quelconque des revendications 15 à 25, dans lequel ledit dispositif comprend en outre au moins un capteur de lumière adapté pour mesurer une intensité de lumière entre ladite source de lumière (22, 22a à 22d) et ledit modulateur spatial de lumière (24),
dans lequel ledit procédé comprend les étapes consistant à

- mettre ledit modulateur de lumière dans un mode non transmissif, et
- pendant que ledit modulateur de lumière est dans ledit mode non transmissif, faire pulser ladite au moins une source de lumière (22, 22a - 22d) pour mesurer une luminosité de ladite source de lumière (22).

**27.** Le procédé selon l'une quelconque des revendications 15 à 26, dans lequel ledit microscope comprend au moins un capteur de lumière (58a à 58d, 58) et comprenant les étapes consistant à

surveiller une luminosité de la source de lumière (22, 22a - 22d) au moyen dudit capteur de lumière (58a - 58d, 58) et générer des paramètres d'étalonnage et
utiliser les paramètres d'étalonnage pour commander la source de lumière (22, 22a - 22d).

**28.** Le procédé selon la revendication 27, dans lequel ledit microscope comprend plusieurs sources de lumières (22a à 22d), dans lequel ledit procédé comprend l'étape consistant à effectuer des mesures d'étalonnage en dehors d'une phase d'intégration de ladite caméra (16), dans lequel lesdites mesures d'étalonnage comprennent les étapes consistant à

- placer ledit modulateur spatial de lumière (24) dans une configuration non transmissive et
- allumer exactement une des sources de lumières (22a à 22d) et mesurer un signal de luminosité au moyen d'un capteur de lumière (58),

et en particulier dans lequel lesdites sources de lumières (22a à 22d) ont des couleurs différentes et dans lequel ledit procédé comprend en outre l'étape consistant à utiliser les mesures d'étalonnage pour les sources de lumière (22a à 22d) afin de maintenir une luminosité relative souhaitée entre au moins deux des sources de lumière (22a à 22d) lorsque lesdites deux sources de lumière sont allumées simultanément pendant l'enregistrement d'une image.

**Fig. 1**

**Fig. 4**

**Fig. 2**

**Fig. 3**

**EP 4 051 083 B1**

**Patent documents cited in the description**

- EP 2549913 A
- US 6072623 A
- JP 2004255177 A
- US 6507434 B